# FASCICULE DE BREVET EUROPEEN

(11) **EP 3 256 836 B1**
(45) Date de publication et mention de la délivrance du brevet: **19.05.2021**
(21) Numéro de dépôt: 16707905.2
(22) Date de dépôt: 11.02.2016
(51) Int. Cl.: G01N 1/10, G01N 33/18, G01N 33/551, G01N 33/543

(54) **DISPOSITIF ET PROCEDE DE CAPTAGE D'AU MOINS UNE ESPÈCE CHIMIQUE COMPRENANT UN CAPTEUR CHIMIQUE ET PROCÉDÉ DE FABRICATION D'UN TEL CAPTEUR CHIMIQUE**
VORRICHTUNG UND VERFAHREN ZUR DETEKTION VON MINDESTENS EINER CHEMISCHEN SPEZIES, EINSCHLIESSLICH EINES CHEMISCHEN SENSORS UND VERFAHREN ZUR HERSTELLUNG SOLCH EINES CHEMISCHEN SENSORS
DEVICE AND METHOD FOR DETECTING AT LEAST ONE CHEMICAL SPECIES, INCLUDING A CHEMICAL SENSOR, AND METHOD FOR MANUFACTURING SUCH A CHEMICAL SENSOR

(30) Priorité: 13.02.2015 FR 1551224
(43) Date de publication de la demande: 20.12.2017
(73) Titulaire: Institut De Recherche Pour Le Développement (IRD), 13002 Marseille 2 (FR)
(72) Inventeur: POINT, David, 33138 Lanton (FR); GAUTIER, Anthony, 37390 Notre Dame d'Oé (FR)
(74) Mandataire: Plasseraud IP
(86) Numéro de dépôt international: PCT/FR2016/050317
(87) Numéro de publication internationale: WO 2016/128686

(56) Documents cités:
- EP-A1- 0 713 576
- EP-A1- 2 484 630
- CASILLI S ET AL: "Piezoelectric sensor functionalised by a self-assembled bipyridinium derivative: characterisation and preliminary applications in the detection of heavy metal ions", BIOSENSORS AND BIOELECTRONICS, ELSEVIER BV, NL, vol. 20, no. 6, 15 décembre 2004 (2004-12-15), pages 1190-1195, XP004648763, ISSN: 0956-5663, DOI: 10.1016/J.BIOS.2004.04.028
- DENISE P RUYS ET AL: "Mercury detection in air using a coated piezoelectric sensor", ANALYTICA CHIMICA ACTA, vol. 404, no. 1, 1 janvier 2000 (2000-01-01), pages 95-100, XP055227851, NL ISSN: 0003-2670, DOI: 10.1016/S0003-2670(99)00673-X
- SABRI Y M ET AL: "Mercury diffusion in gold and silver thin film electrodes on quartz crystal microbalance sensors", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, vol. 137, no. 1, 28 mars 2009 (2009-03-28) , pages 246-252, XP025962274, ISSN: 0925-4005, DOI: 10.1016/J.SNB.2008.11.032 [extrait le 2008-12-03]
- RODRIGUEZ GUTIERREZ J A ET AL: "Development of ionoselective electrochemical sensors by using the sol-gel process", ANALYTICA CHIMICA ACTA, ELSEVIER, AMSTERDAM, NL, vol. 524, no. 1-2, 25 octobre 2004 (2004-10-25), pages 339-346, XP004582530, ISSN: 0003-2670, DOI: 10.1016/J.ACA.2004.02.066

## Description

L'invention a trait au domaine de la mesure de la concentration d'espèces chimiques dans un fluide, et plus particulièrement au domaine de la mesure de la concentration des espèces inorganiques, moléculaires ou biologiques dans une étendue d'eau.

Les activités humaines nécessitent la surveillance en polluants des écosystèmes, pour des raisons à la fois de santé et d'études d'impact de l'environnement. Ces polluants peuvent être produits naturellement dans les écosystèmes (ex. biotoxines) ou issus eux-mêmes des activités humaines. Parmi les polluants les plus problématiques à ces égards, on peut citer les éléments traces métalliques, et plus particulièrement le mercure. Ainsi, l'invention trouvera une application particulière, mais non restreinte, à la détection des formes toxiques de mercure.

Les sources naturelles en mercure sont relativement nombreuses. On peut citer à titre d'exemples les émissions volcaniques dégageant du mercure et les émissions passives vers l'atmosphère des océans ou des étendues d'eau continentales. Les activités humaines impliquant l'utilisation de combustible fossile génèrent également du mercure vers l'atmosphère. Ainsi, le mercure se trouve dans l'environnement en quantités non négligeables.

Or, le mercure présente des propriétés physico-chimiques et des niveaux de toxicité qui dépendent de ses différentes formes chimiques. Notamment, le mercure de l'air retombant dans l'eau (rivières, lacs, mers, océans, etc.) peut être converti en composé organométallique, tel que le méthylmercure (noté MeHg), qui est la forme la plus toxique des composés organométalliques du mercure. Le caractère toxique du MeHg a amené les autorités sanitaires de nombreux pays à définir des seuils de concentrations à ne pas dépasser dans les aliments, tels que les poissons.

Les mécanismes à la base de la formation du MeHg, par un processus appelé méthylation du mercure, sont relativement peu connus. En effet, dans les milieux aquatiques, le mercure se retrouve sous différentes formes, dont le mercure élémentaire dissout et le mercure inorganique qui sous chacune de ces formes sont susceptibles de se transformer en MeHg. On estime qu'environ 1 à 30% du mercure total, suivant les écosystèmes est propre à subir le processus de méthylation. A l'inverse, le MeHg peut subir une déméthylation. Ainsi, la simple mesure brute de la concentration mercure totale ne permet pas de renseigner sur la toxicité du mercure.

Il est donc nécessaire, pour prendre en compte l'ensemble des risques sanitaires et environnementaux dus au mercure, de mesurer ces différentes formes chimiques, leur biodisponibilité, leur complexes avec des contre-ions ou la matière organique, ainsi que leur stabilité.

Il est connu de déterminer la concentration des espèces à analyser en se basant sur les lois de Fick, régissant le phénomène de diffusion entre le milieu ambiant et un support de préconcentration. Notamment, en fonction du temps de diffusion, c'est-à-dire du temps pour capturer les espèces sur un support approprié, et connaissant le coefficient de diffusion, il est possible de relier la quantité d'espèces capturée à leur concentration.

La captation des espèces par diffusion, afin d'appliquer les lois de Fick, est réalisée par exemple à l'aide d'un capteur passif tel que le capteur DGT (pour « Diffusive Gradient in Thin film »).

Le capteur DGT est utilisé de manière générale pour mesurer la quantité d'une espèce dans un milieu liquide, même lorsque cette espèce est en très faible concentration.

Le capteur DGT se présente sous la forme d'un support de préconcentration destiné à être immergé dans un liquide. Dans le support, sont superposées une couche d'un premier gel perméable, dite couche diffusive, et une couche de préconcentration qui est constituée d'un deuxième gel comprenant des particules de résine, dite couche de résine. La couche diffusive comprend une portion de surface découverte par le support, en contact avec le liquide. La taille des pores de la couche diffusive permet de déterminer la taille maximale des espèces qui atteindront la couche de résine. Les espèces à mesurer diffusent à travers la couche diffusive jusqu'à la couche de résine, où elles sont piégées par les particules de résines et où elles se concentrent. En connaissant la surface du capteur, l'épaisseur de la couche diffusive, le coefficient de diffusion des espèces dans le gel, le temps d'immersion du capteur et en mesurant la concentration d'espèces accumulées dans la couche de résine, il est possible de calculer la concentration en espèces dans le milieu liquide à analyser. Plus le temps d'immersion est important, plus la concentration en espèces
piégées est importante. Plus précisément, une fois que le capteur DGT a été immergé pour une durée déterminée, il est récupéré. Les particules de résine sont extraites de la couche de résine et sont analysées pour obtenir la concentration en espèces piégées.

Le document EP 0 713 576 présente le capteur DGT. Comme expliqué dans ce document, l'épaisseur des couches de gel n'est pas forcément la seule dimension à prendre en compte. En effet, en pratique, il se forme à l'interface entre la couche diffusive en contact avec le liquide et le liquide une couche de diffusion intermédiaire, appelée Diffusive Boundary Layer (DBL). La couche DBL correspond à la zone dans le liquide dans laquelle tout analyte piégé par le capteur est renouvelé par le milieu extérieur pour compenser la disparition et ainsi rétablir l'équilibre. L'épaisseur de la couche DBL participe donc au chemin de diffusion total des analytes.

Dans ce document, il est expliqué que, dépendamment du milieu à analyser, la couche DBL peut être négligée ou non. Pour négliger la couche DBL, la couche diffusive doit avoir une épaisseur minimale, de sorte à être très supérieure à celle de la couche DBL. Par exemple, en considérant que dans un milieu agité l'épaisseur de la couche DBL est au maximum de 0,1 mm, en donnant à la couche diffusive une épaisseur de 1 mm, l'épaisseur de la couche DBL est négligeable dans le calcul de la concentration en ions. Cependant, à l'inverse, dans un milieu tel que des eaux stagnantes, la couche DBL peut atteindre 1 mm. Par conséquent, il est nécessaire pour ce type de milieux de prendre en compte cette épaisseur dans le calcul de la concentration en ions.

Or, on le comprend, plus le chemin de diffusion, formé par l'épaisseur de la couche diffusive et de la couche DBL, est long, plus le temps de diffusion, c'est-à-dire le temps pour que les espèces traversent la couche DBL et la couche diffusive et se fixent dans la couche de résine, est important. Dès lors, en imposant une épaisseur minimale à la couche diffusive, il est également imposé un temps minimal d'immersion du capteur pour obtenir une concentration dans la couche de résine qui soit exploitable. En outre, le modèle pour le calcul de la concentration dans le milieu à analyser dépend des conditions dans ce milieu. Notamment, lorsque le courant est faible il faut tenir compte de l'épaisseur de la couche DBL. Se pose alors la question de la détermination de cette épaisseur, avec les erreurs dues aux approximations qui en résultent.

Le document EP 2 484 630 A1 décrit un nano capteur d'ions métalliques lourds dans un fluide. Le nano capteur comprend en particulier une membrane cylindrique présentant une électrode d'or sur chaque face, ainsi que des contacts piézoélectriques permettant, sous tension alternative, de générer une agitation sonique favorisant la diffusion à travers la membrane.

Dans le document « Piezoelectric sensor functionalised by a self-assembled bipyridinium derivative : characterisation and preliminary applications in the détection of heavy metal ions », CASILLI S ET AL, la nature des espèces chimiques d'un milieu à analyser au contact d'une électrode est déterminée en mesurant la fréquence des vibrations d'un cristal plongé dans le milieu.

De même, dans le document « Mercury détection in air using a coated piezoelectric sensor », DENISE P RUYS ET AL, la fréquence des vibrations d'un cristal permet de déterminer la concentration de mercure dans le milieu à analyser.

Il existe donc un besoin pour un nouveau système de détection d'une substance chimique surmontant notamment les inconvénients précités.

A cet effet, selon un premier objet, l'invention propose un dispositif de captage d'au moins une espèce chimique dans un milieu à analyser comprenant un capteur chimique, selon la revendication 1.

Le capteur chimique comprend :
- un substrat,
- un support fonctionnalisé destiné à capturer et à accumuler l'espèce chimique, et fixé sur une face du substrat.

Le dispositif comprend en outre des moyens de diffusion entre le milieu à analyser et le support fonctionnalisé. Les moyens de diffusion comprennent un système générateur de vibrations connecté au capteur chimique pour soumettre le capteur chimique à des vibrations contrôlées.

Le dispositif de captage est ainsi d'être le siège d'un phénomène de diffusion contrôlée par les vibrations. Or, la diffusion est nécessaire au dispositif de captage, car elle permet de déduire la concentration en l'espèce chimique visée. Grâce au contrôle de la diffusion, il est possible d'obtenir une mesure de concentration avec une précision accrue, et pour une durée de mesure qui est réduite comparativement à l'état de la technique.

Le dispositif peut comprendre les caractéristiques suivantes, seules ou en combinaison :
- les moyens de diffusion comprennent une couche diffusive superposée au support fonctionnalisé, la couche diffusive procurant une sélection des espèces chimiques à diffuser ;
- de la couche diffusive est de préférence inférieure à 1 micromètre, voire est comprise entre 250 nm et 10 nm, de sorte que la diffusion à travers elle se fait plus rapidement que dans l'état de la technique ;
- la couche de diffusion est en gel d'Agarose, particulièrement bien adaptée au captage des espèces chimiques du mercure ;
- l'épaisseur du support fonctionnalisé est inférieure à 1 micromètre voire inférieure à 1 nm ;
- le substrat comprend deux faces opposées sur chacune desquelles est adhérisée un support fonctionnalisé, de manière à doubler, sur un même substrat, les surfaces de diffusion ;
- le système générateur de vibrations comprend un capteur de vibration relié au capteur chimique, qui permet de contrôler les vibrations effectivement transmises au capteur chimique ;
- le système générateur de vibrations comprend un micromoteur vibrant à haute fréquence connecté au capteur chimique et des moyens de contrôle du micromoteur ;
- le dispositif comprend d'une part un boîtier de commande dans lequel les moyens de contrôle du micromoteur sont agencés, et d'autre part une tête chimique comprenant le capteur chimique et le système générateur de vibrations. Le boîtier de commande et la tête chimique sont amovibles l'un par rapport à l'autre et comprenant chacun au moins un embout de connexion pour les assembler l'un avec l'autre. La tête chimique peut ainsi être remplacée aisément après chaque mesure, tout en conservant le boîtier ; et
- le dispositif de captage se présente sous la forme d'un boîtier unique.

Un deuxième objet de l'invention est de proposer un procédé de détermination de la concentration d'au moins une espèce chimique dans un milieu à analyser au moyen d'un dispositif de captage selon la revendication 14. Le procédé comprend, les étapes suivantes :
- mettre le capteur chimique en contact avec le milieu à analyser ;
- soumettre le capteur chimique à des vibrations de fréquences contrôlées par le système générateur de vibrations ;
- capter l'espèce chimique à analyser par le support fonctionnalisé par diffusion entre le milieu à analyser et le support fonctionnalisé ;
- au bout d'une durée déterminée, récupération du capteur chimique hors du milieu à analyser ;
- extraction de l'espèce chimique piégée dans le capteur chimique ;
- en utilisant le principe de diffusion et la durée déterminée, détermination de la concentration de l'espèce chimique à analyser.

La fréquence des vibrations est comprise entre 2 et 1000 Hz, voire entre 10 et 600 Hz, afin d'optimiser le phénomène de diffusion. Les vibrations peuvent être réglées en fonction de l'agitation déjà présente dans le milieu à analyser.

Selon un exemple de réalisation du procédé, au moins une espèce chimique est le méthylmercure, espèce particulièrement toxique pour l'homme.

Un troisième aspect de l'invention est un dispositif de captage selon la revendication 13, dans lequel le capteur est fabriqué par un procédé comprenant les étapes suivantes :
- décapage d'au moins une face d'une plaque formant le substrat,
- adhérisation d'un support fonctionnalisé sur la face décapée du substrat, le support fonctionnalisé ayant une épaisseur inférieure à 1 micromètre ;
- dépôt par spin-coating, ou dip coating d'une couche diffusive sur le support fonctionnalisé, la couche diffusive ayant une épaisseur inférieure à 1 micromètre.

D'autres avantages et applications apparaîtront à la lumière de la description accompagnée des figures, dans lesquelles :
- la figure 1 est une représentation schématique d'un exemple de réalisation d'un dispositif de captage d'au moins une substance chimique ;
- la figure 2 est une vue schématique d'un capteur chimique du dispositif de la figure 1 ;
- la figure 3 est une vue éclatée du dispositif de captage de la figure 1 selon un mode de réalisation ;
- la figure 4 est une vue d'un boîtier, élément du dispositif de captage de la figure 3 ;
- la figure 5 est une vue d'une extrémité du boîtier de la figure 4 ;
- la figure 6 est une vue du capteur chimique sur un support ;
- la figure 7 est une vue éclatée du dispositif de captage en deux parties, un boîtier de contrôle et une tête chimique ;
- la figure 8 est une vue de côté de la tête chimique de la figure 7 ;
- la figure 9 est une vue du dispositif de captage assemblé ;
- la figure 10 est un graphique comparant les performances du dispositif des figures précédente et d'un dispositif de type DGT de l'état de la technique.

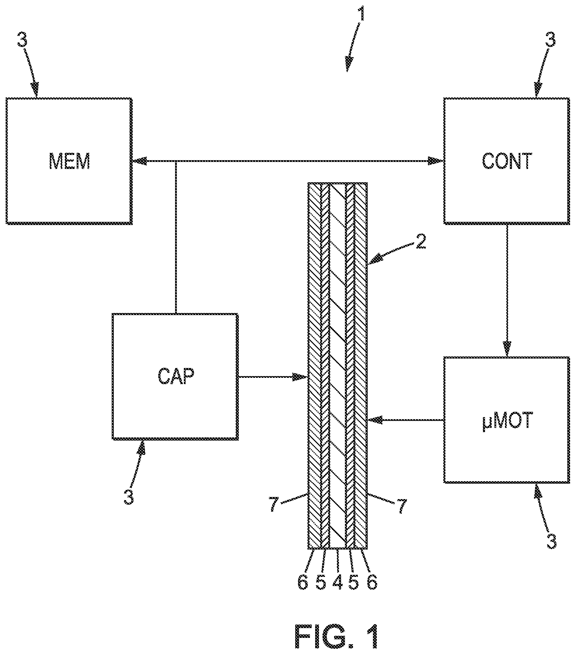

Sur la figure 1, il est représenté de manière schématique un exemple de réalisation d'un dispositif 1 de captage d'au moins une espèce chimique. Par substance chimique, on entend dans ce qui suit tout type d'espèce au sens large, notamment les polluants dans un milieu donné, tels que des éléments traces métalliques et leurs complexes, des molécules, mais également des espèces biologiques, telles que des fragments d'ADN.

Le dispositif 1 de détection comprend au moins un capteur 2 chimique, c'est-à-dire capable de piéger au moins une espèce chimique. Dans ce qui suit, mais de manière non limitative, il sera considéré que l'espèce chimique à capturer est un composé du mercure, notamment le MeHg. Toutefois, en pratique, le capteur 2 est destiné à capturer plusieurs espèces, tels que plusieurs composés du mercure susceptibles de subir une méthylation. Le dispositif 1 de captage comprend par ailleurs des moyens de diffusion entre le milieu à analyser et le capteur 2 chimique. Les moyens de diffusion sont définis comme étant tout moyen grâce auquel un phénomène de diffusion contrôlée se produit. En effet, comme cela sera explicité plus loin, le principe du capteur 2 chimique est basé sur le phénomène de diffusion, permettant de déduire la concentration en l'espèce chimique dans le milieu à analyser. L'utilisation du capteur 2 chimique implique donc la mise en place d'un phénomène de diffusion de manière contrôlée. A cet effet, les moyens de diffusion comprennent un système 3 générateur de vibrations, connecté au capteur 2 chimique, de manière à soumettre le capteur 2 chimique à des vibrations contrôlées.

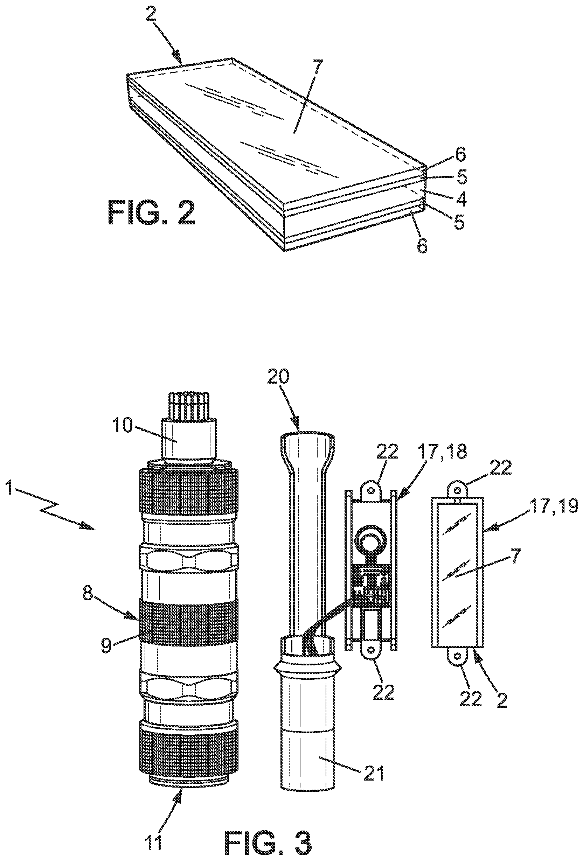

Selon l'exemple illustré sur les figures, le capteur 2 chimique se présente sous la forme d'une plaque mince, comprenant un substrat **4** tel qu'une plaque de verre. La plaque 4 de verre présente deux faces opposées, dont au moins une est fonctionnalisée comme cela sera explicité plus loin. En variante, et telles qu'illustrées sur les figures 1 et 2, les deux faces opposées de la plaque 4 de verre peuvent être fonctionnalisées. Plus précisément, une couche appelée support 5 fonctionnalisé est liée sur au moins une face du substrat 4. Le support 5 fonctionnalisé est défini comme présentant deux fonctions. Une première fonction est de se lier à une face du substrat 4. La deuxième fonction est de capter l'espèce à analyser et de l'accumuler. Par exemple, pour capter un composé du mercure sous forme liquide, le support 5 fonctionnalisé peut comprendre des groupements fonctionnels de type thiol fixés au support en verre via des liaisons organosilanes. Pour capturer du mercure sous forme gazeuse et la plupart des autres formes chimiques dissoutes du Hg, le support 5 fonctionnalisé peut être plutôt une couche d'or, liée au substrat par coating, et capturant le mercure par amalgamation.

De manière plus générale, la nature du substrat 4 et du support 5 fonctionnalisé est choisie en fonction de la ou des espèces chimiques à capturer.

Sur chaque support 5 fonctionnalisé, il peut être superposé une couche 6 diffusive, destinée à favoriser l'adsorption de la substance chimique en question et à la conduire jusqu'au support 5 fonctionnalisé par le phénomène de diffusion. Par exemple, dans le cas où il est cherché à piéger du mercure, la couche 6 diffusive peut comprendre un gel d'Agarose. La couche 6 diffusive comprend des pores dont la taille peut être variée en fonction de l'analyse qu'il est cherché à effectuer. Par exemple, toujours dans l'exemple du mercure, lorsqu'il est cherché à piéger les espèces labiles de MeHg et le mercure inorganique, les pores sont de préférence au maximum de 20 nm (nanomètre). Lorsqu'il est cherché à piéger en plus les espèces plus complexes, la taille de spores est augmentée, par exemple jusqu'à 450 nm. La couche 6 diffusive présente ainsi à son tour une double fonction : assurer la diffusion des espèces en son sein, et sélectionner une taille maximale d'espèces pouvant diffuser. Chaque couche 6 diffusive présente alors une surface **7** supérieure libre, apte à être en contact avec le milieu à analyser. Comme cela sera explicité plus loin, la couche 6 diffusive est facultative. Dans ce qui suit, il est considéré l'exemple d'un capteur 2 avec une couche 6 diffusive.

Le capteur 2 chimique a alors une forme générale parallélépipédique, dont deux faces opposées sont formées par les surfaces 7 libres des couches 6 diffusive. Les dimensions du capteur 2 chimique sont, selon un exemple de réalisation de 76 mm (millimètres) sur 26 mm, pour une épaisseur, formée par la superposition des couches 5, 6 sur le substrat 4, d'environ 1 mm. La surface disponible du capteur 2 chimique pour être en contact avec le milieu analyser peut comprendre une ou les deux faces 7 libres.

Le système 3 générateur de vibrations comprend par exemple un micromoteur **µMOT,** lequel est connecté au capteur 2 chimique et des moyens **CONT** de contrôle du micromoteur µMOT. Les moyens CONT de contrôle comprennent notamment un microcontrôleur et un générateur de tension permettant de contrôler la fréquence et l'intensité des vibrations du micromoteur µMOP en modifiant sa tension d'alimentation. Le système 3 générateur de vibrations peut comprendre en outre d'autres instruments permettant de surveiller le fonctionnement du dispositif 1. Par exemple, le système 3 de vibrations comprend un capteur **CAP** de vibrations, qui est connecté au capteur 2 chimique, afin d'enregistrer la fréquence à laquelle le capteur 2 chimique vibre effectivement. Typiquement, le capteur CAP est un accéléromètre. Les informations issues du capteur CAP de vibrations sont traitées par les moyens CONT de contrôle et enregistrées dans une mémoire **MEM.** La correction de la fréquence de vibration se fait de préférence en temps réel, grâce aux moyens CONT de contrôle qui analysent la fréquence de vibration fournie par l'accéléromètre et régule en fonction la tension d'alimentation du micromoteur µMOT.

Le système 3 de générations comprend également des moyens de commande, afin d'enclencher, d'arrêter ou de programmer les vibrations. Selon un premier exemple, le système 3 générateur de vibrations peut être déclenché ou arrêté manuellement, par exemple en appuyant sur un interrupteur accessible à l'extérieur du dispositif 1. Selon un deuxième exemple, le système 3 générateur de vibrations peut être déclenché ou arrêté à distance. Dans ce cas, les moyens CONT de contrôle comprennent des moyens de réception d'un signal de commande. Un dispositif externe peut être utilisé pour générer et émettre le signal de commande à destination des moyens de réception. Un tel dispositif peut par exemple être un appareil de type Smartphone. Les vibrations peuvent par ailleurs être programmées, pour se déclencher à un ou plusieurs moments donnés et pour une ou plusieurs périodes données.

La fréquence des vibrations est comprise entre 2 Hz et 1000 Hz, et de préférence entre 20 et 600 Hz.

Le dispositif 1 de captage peut se présenter par exemple sous une forme générale de tube, permettant une manipulation aisée. Plus précisément, selon un mode de réalisation, le dispositif 1 de captage peut être décomposé en deux parties.

Une première partie, appelée boîtier 8 de commande, intègre par exemple les moyens CONT de contrôle et la mémoire MEM du système 3 générateur de vibration. Le boîtier 8 de commande comprend une enveloppe 9 cylindrique étanche, réalisée par exemple en matière plastique, dans laquelle les composants CONT et MEM sont logés. Une alimentation, par exemple une batterie, peut également être placée dans le boîtier 8. Le boîtier 8 de commande est muni à une première extrémité d'un embout 10 de connexion étanche. A sa deuxième extrémité, le boîtier 8 de commande comporte un logement **11** pour un ensemble **12** de capteurs complémentaires, permettant de fournir des données complémentaires sur l'environnement afin d'améliorer la connaissance de l'environnement et par là le traitement ultérieur des mesures par le dispositif 1. Par exemple, l'ensemble 12 de capteurs complémentaires peut comprendre un capteur **13** de luminosité et un capteur **14** de température et de pression du milieu dans lequel le dispositif 1 est immergé. Eventuellement, un ensemble **15** de voyants lumineux peut être placé dans le logement 11 afin de fournir une indication visuelle sur l'état du dispositif 1 (en fonctionnement, à l'arrêt, batterie faible, etc). L'ensemble 12 de capteurs complémentaires placé dans le logement 11 du boîtier 8 de commande est recouvert d'une résine transparente et imperméable, afin de les protéger tout en permettant la prise de mesure. Le boîtier 8 peut en outre loger des moyens pour déterminer la position et les changements de position du dispositif 1. Par exemple, ces moyens peuvent être un module GPS afin de déterminer la localisation géographique du dispositif 1, et ses déplacements éventuels. Ces moyens peuvent également être un ensemble accéléromètre et gyroscope, permettant de déterminer l'orientation du dispositif 1, par exemple si le dispositif 1 est bien horizontal ou vertical lors de la durée de son immersion dans le milieu à analyser. La connaissance de l'orientation du dispositif 1 permet de corriger éventuellement la commande des vibrations du dispositif 1. Des moyens peuvent également être prévus pour connaître la profondeur à laquelle le dispositif se trouve immergé. Les mesures de l'ensemble 12 de capteurs complémentaires, et celles relatives à sa position ou les changements de position, sont alors enregistrées dans la mémoire MEM du système 3 générateur de vibrations. Un bouchon 16 peut être rapporté sur le boîtier 8 de commande pour recouvrir le logement 11 et l'ensemble 12 de capteurs complémentaires afin notamment de le protéger lors du transport du dispositif. Ce bouchon 16 est retiré lorsque le dispositif est plongé dans le milieu à analyser.

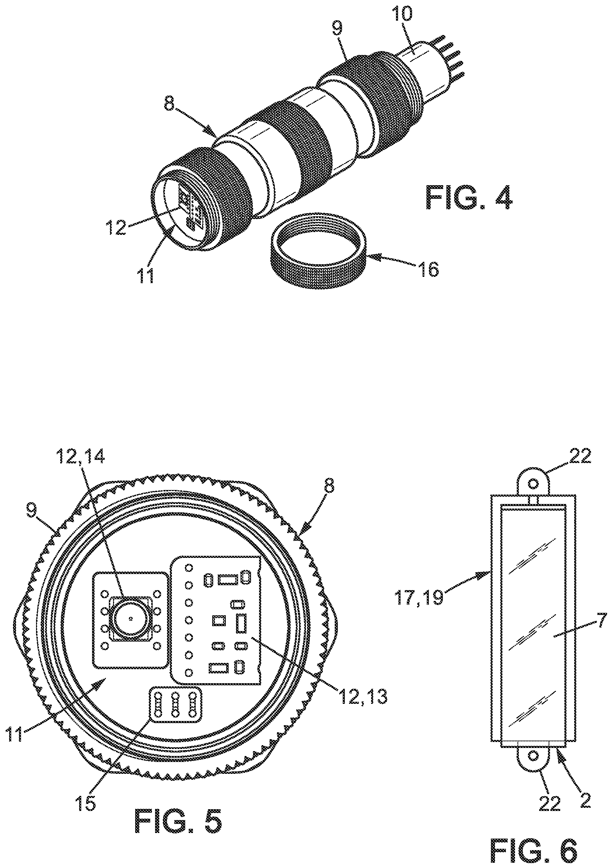

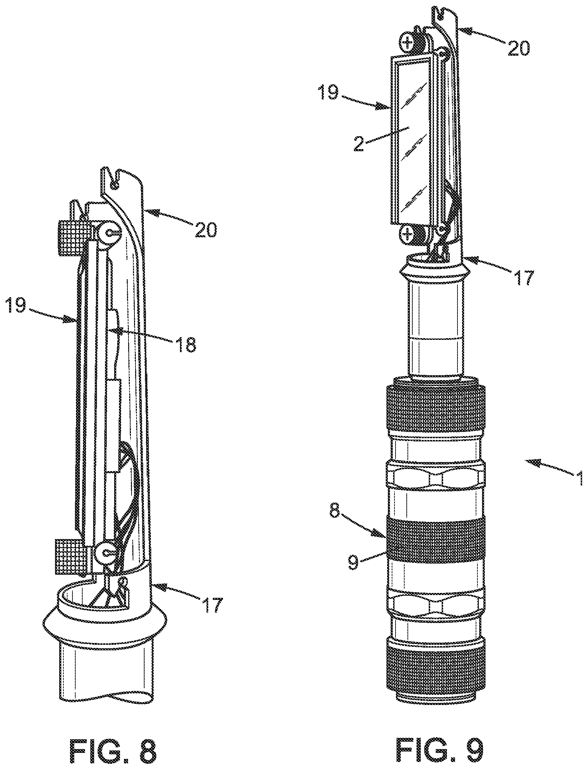

La deuxième partie est appelée la tête **17** chimique et comprend le capteur 2 chimique, le micromoteur µMOT et le capteur CAP de vibrations. Plus précisément, la tête 17 chimique comprend un premier support **18,** en forme de plaque rectangulaire, par exemple réalisé en plastique. Le premier support 18 comprend des logements pour le micromoteur µMOT et pour le capteur CAP de vibration. Un gel de silicone est déposé par-dessus l'ensemble, de sorte que le micromoteur µMOT et le capteur CAP de vibrations sont maintenus en place sur le premier support 18. La tête 17 chimique comprend un deuxième support **19** pour le capteur 2 chimique. Le deuxième support 19 se présente également sous la forme d'une plaque rectangulaire de dimensions sensiblement égales à celle du premier support 17, et qui peut être réalisée en plastique, et forme un logement pour le capteur 2 chimique. Des moyens de clipsage de collage, ou de retenue par aimantation peuvent être prévus pour maintenir le capteur 2 chimique et son deuxième support 19 au support 18. Selon le mode de réalisation illustré sur les figures 3 et 6 à 9, le capteur 2 chimique présente une unique surface 7 libre supérieure de la couche 6 de diffusion permettant de capter les espèces chimiques. Enfin, la tête 17 chimique comprend un support 20 commun, permettant d'y assembler le premier support 18 muni du micromoteur µMOT et du capteur CAP de vibrations ainsi que le deuxième support 19 muni du capteur 2 chimique. Le support 20 commun, également en plastique, comprend par ailleurs un embout **21** de connexion étanche, complémentaire de l'embout 10 de connexion du boîtier 8 de commande. Par exemple, l'embout 10 de connexion du boîtier 8 de commande comprend des broches complémentaires de récepteurs sur l'embout 21 de connexion de la tête 17 chimique.

L'assemblage de la tête 17 chimique se fait de la manière suivante.

Le micromoteur µMOT et le capteur CAP de vibrations sont tous les deux connectés électroniquement, au moyen de fils, à l'embout 21 de connexion de la tête 17 chimique.

Le capteur 2 chimique est mis en place sur le deuxième support 19, qui est ensuite fixé au premier support 18. A cet effet, le premier support 18 et le deuxième support 19 sont tous les deux munis d'œillères **22** trouées, permettant leur fixation ensemble au moyen de vis ou d'un système de retenue magnétique. Le contact entre le premier support 18 et le deuxième support 19 est réalisé sur la plus grande surface possible.

Les deux supports 18, 19 sont alors fixés sur le support 20 commun à l'aide d'élastiques, non représentés sur les figures. A cet effet, le premier support 18 par exemple comprend des crochets disposés à proximité des ses quatre coins sur chacun desquels un élastique peut être accroché. De même, le support 20 commun comprend des crochets pour les élastiques. La raideur des élastiques est contrôlée, de sorte que les élastiques font office d'amortisseurs, en limitant la transmission des vibrations du micromoteur µMOT au support 20 commun et donc au reste du dispositif 1.

Ainsi, la quasi-totalité des vibrations générées par le micromoteur µMOT sont transmises au capteur 2 chimique, par l'intermédiaire de la large surface de contact entre les supports 18, 19.

La tête 17 chimique ainsi constituée peut alors être connectée au boîtier 8 de commande par l'enfichage de leur embout 10, 21 de connexion respectif. Les embouts 10, 21 de connexion assurent une connexion étanche.

Enfin, un capuchon amovible, non représenté, peut venir recouvrir la tête 17 chimique afin de la protéger lors des étapes de transport et de stockage. Le capuchon est retiré lorsque le dispositif 1 est plongé dans le milieu à analyser.

Les pièces en plastique, notamment l'enveloppe 9 du boîtier 8, le bouchon 16 et le capuchon et les supports 18, 19 et 20, peuvent être réalisés par exemple par moulage ou par impression 3D.

Le mode de réalisation ainsi décrit comprend un unique capteur 2 chimique, présentant une seule face 7 libre supérieure. Toutefois, le dispositif pourra comprendre plusieurs capteurs 2 chimiques mis en vibrations par un même micromoteur. Par exemple, le dispositif peut comprendre deux capteurs 2 chimiques, présentant chacun une seule face en contact avec le milieu à analyser, et chacun fixé sur un support similaire au deuxième support 19 décrit, mais disposé de part et d'autre du premier support 18, de sorte qu'un même micromoteur µMOT peut mettre en vibrations les deux capteurs 2 chimiques.

Ainsi, le dispositif 1 de captage est facilement transporté jusqu'au lieu des déploiements in situ, par exemple au bord d'une rivière, en mer ou de toute étendue d'eau. Préalablement, le dispositif 1 peut être programmé en le connectant à un système informatique, notamment par l'intermédiaire de l'embout 10 de connexion du boîtier 8, avant assemblage avec la tête 17 chimique. Cette connexion peut également être utilisée pour charger les batteries du dispositif dans le boîtier 8. Lorsque le dispositif 1 doit être utilisé, le capuchon et le bouchon 16 sont retirés, et le dispositif 1 est plongé dans l'eau. Le système 3 générateur de vibrations est alors mis en fonctionnement, afin de transmettre les vibrations au capteur 2 chimique. Les moyens de commande peuvent prévoir que les vibrations appliquées au capteur 2 chimique varient dans le temps. Les espèces à analyser diffusent depuis le milieu à analyser vers le support 5 fonctionnalisé, sur ou dans lequel elles s'accumulent. Le temps d'accumulation sur le support 5 fonctionnalisé est contrôlé par la dynamique de vibration. En effet, en absence de vibrations, il peut être considéré que quasiment aucune diffusion n'a lieu. Ainsi, en programmant la période pendant laquelle le système 3 générateur de vibrations est enclenché, le temps de diffusion est également contrôlé. En d'autres termes, la commande de l'enclenchement et de l'arrêt du générateur 3 de vibrations peut constituer un interrupteur pour le phénomène de diffusion au sein du ou des capteurs 2 chimiques.

Puis, le dispositif 1 est récupéré; le capuchon et le bouchon 16 sont de nouveau mis en place, et le dispositif 1 peut être ramené par exemple en laboratoire pour analyser les espèces piégées. La tête 17 chimique peut être déconnectée du boîtier 8 afin de faciliter le traitement du capteur 2 chimique. Les données enregistrées dans la mémoire MEM dans le boîtier 8, telles que les données de l'ensemble 12 de capteurs complémentaire et l'historique des vibrations appliquées au capteur 2 chimique et des ses positions, peuvent alors être facilement récupérées. Par exemple, l'embout 10 de connexion du boîtier 8 est utilisé pour connecté le dispositif à un système informatique. Le capteur 2 chimique est désassemblé de la tête 17 chimique afin d'être analysé. Par exemple, un procédé d'élution peut être mis en place sur le capteur 2 chimique afin de récupérer les espèces capturées et de les analyser. Pour chaque prélèvement in situ, le support 5 fonctionnalisé est remplacé en changeant le capteur 2 chimique.

Une fois que les espèces capturées sont récupérées, il en est déduit la concentration en ces espèces dans le milieu à analyser, avec une précision accrue comparé aux dispositifs de l'état de la technique. En effet, comme expliqué ci-dessus, le calcul de la concentration est basé sur les équations de Fick. De manière classique, des expériences sont menées en laboratoire afin d'établir des modèles pour appliquer les équations de Fick. Avec les capteurs de type DGT de l'état de la technique, les modèles développés en laboratoire ne collent pas à la réalité du terrain, puisque les conditions d'agitation au sein du milieu à analyser ne peuvent être qu'approchées par les modèles en laboratoire. Par exemple, le coefficient de diffusion déterminé en laboratoire peut ne pas correspondre à celui du terrain, du fait de la présence de la couche DBL dont l'épaisseur est inconnue. Grâce au dispositif 1 de captage comprenant le système 3 générateur de vibrations, les vibrations appliquées au capteur 2 chimique lors des mesures dans le milieu à analyser sont identiques à celles appliquées en laboratoire. En particulier, le capteur CAP de vibrations mesure les vibrations réellement subies par le capteur 2 chimique et permet alors d'exercer un rétrocontrôle tenant compte de l'agitation du milieu dans lequel le dispositif 1 est immergé, de sorte que les vibrations réellement appliquées au capteur 2 chimique sont connues et le modèle à appliquer peut être choisi en conséquence.

De plus, grâce à la mise en vibration du capteur 2 chimique par le micromoteur µMOT, l'épaisseur de la couche DBL (« Diffusive Boundary Layer ») présentée en introduction est contrôlée. En effet, l'agitation créée par les vibrations autour du capteur 2 chimique agit sur le renouvellement des espèces à proximité des faces 7 supérieures des couches 6 diffusives. Ainsi, plus les vibrations sont importantes, plus le renouvellement se fait rapidement, et plus l'épaisseur de la couche DBL est faible. Ce contrôle apporte de nombreux avantages, et notamment la réduction du temps de diffusion, c'est-à-dire du temps que mettent les espèces pour passer du milieu à analyser au support 5 fonctionnalisé.

Grâce au contrôle de l'épaisseur de la couche DBL, son influence sur le phénomène de diffusion est connue, de sorte qu'il n'est pas nécessaire de mettre en place une épaisseur minimale de la couche 6 diffusive. Plus précisément, on peut définir un chemin de diffusion comme étant l'épaisseur traversée par les espèces à analyser et au sein de laquelle se produit le phénomène de diffusion entre le milieu à analyser et le support 5 fonctionnalisé. Ainsi, en d'autres termes, le chemin de diffusion est défini comme étant la somme de l'épaisseur de la couche DBL et l'épaisseur de la couche 6 diffusive. Comme l'épaisseur de la couche 6 diffusive peut être considérablement réduite, le chemin de diffusion, et par là le temps de diffusion peuvent être considérablement réduits.

A l'extrême, la couche 6 diffusive peut être supprimée, de sorte que la couche DBL seule suffit à être le siège du phénomène de diffusion. Cependant, comme indiqué précédemment, la mise en œuvre par la couche 6 diffusive d'une sélection sur la taille des espèces à capturer peut être souhaitable. Selon un mode de réalisation, l'épaisseur de la couche 6 de résine est inférieure au micromètre, voire inférieure à 100 nanomètres.

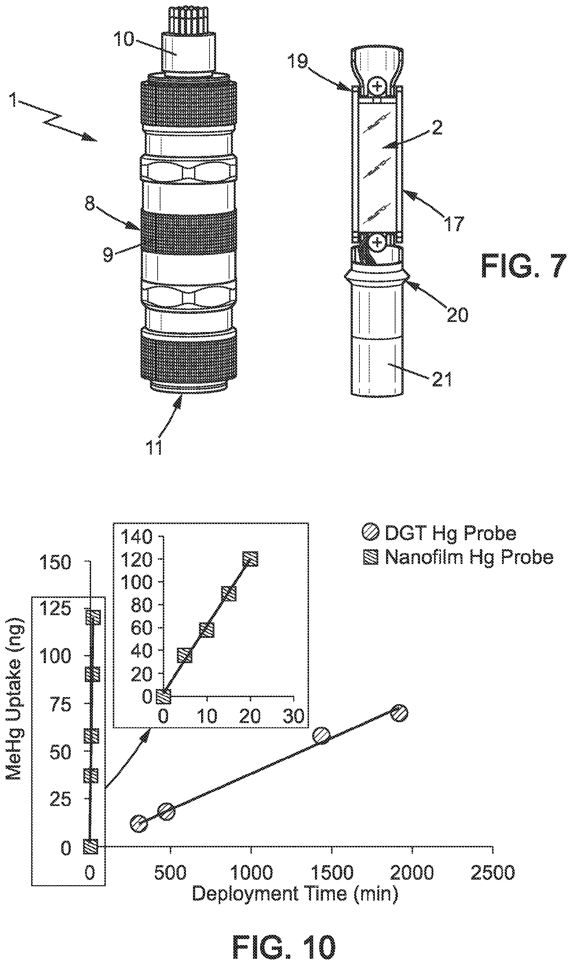

Pour comparer les vitesses de diffusion entre le capteur DGT de l'état de la technique et le dispositif 1 de captage, il a été mesuré la quantité de MeHg capturée par un capteur chimique de type DGT de l'état de la technique (« Capteur DGT Hg»), sans système générateur de vibrations, et par le dispositif 1 selon l'invention (« Capteur Nanofilm Hg »), avec un générateur 3 de vibrations, en l'absence de couche 6 diffusive, en fonction du temps. La figure 10 reporte les résultats pour les deux dispositifs, la quantité de MeHg étant mesurée en nanogramme et le temps en minutes.

Pour le dispositif DGT, la droite rapportant la quantité de MeHg capturée en fonction du temps présente un coefficient directeur très inférieur à 1, de l'ordre de 0,03. Le temps de diffusion se mesure en heures voire en jours.

Au contraire, pour le dispositif selon l'invention, la droite rapportant la quantité de MeHg capturée en fonction du temps présente un coefficient directeur supérieur à 1, de l'ordre de 5, le temps de diffusion se mesurant en minutes.

Ainsi, la vitesse de diffusion du dispositif DGT de l'état de la technique est très inférieure à celle du dispositif 1 selon l'invention. Il a été mesuré que pour une même quantité de MeHg capturé, la diffusion au sein le dispositif selon l'invention se fait 160 fois plus vite que celle au sein d'un dispositif selon l'état de la technique. L'ajout du système 3 générateur de vibrations au dispositif 1 selon l'invention permet ainsi d'obtenir très rapidement une quantité de MeHg exploitable pour effectuer des mesures de concentration.

On va maintenant décrire un exemple de procédé de fabrication du capteur 2 chimique permettant d'atteindre de faibles épaisseurs pour les couches 5, 6.

Dans une première étape, une plaque est décapée sur au moins une de ses faces, en pratique sur deux faces opposées, pour former le substrat 4. Ainsi, lorsque le substrat est en verre, le décapage permet de libérer des liaisons oxygène - hydrogène (liaison OH). Le support 5 fonctionnalisé est ensuite appliqué en couche sur la face décapée. Le substrat étant en verre, le support 5 fonctionnalisé adhère au substrat en formant par exemple une liaison organosilane avec le verre et forme une couche d'environ 0,7 nm d'épaisseur. Enfin, accessoirement, la couche 6 de diffusion est déposée sur le support 5 fonctionnalisé par un procédé de spin-coating, ou dip-coating, permettant d'atteindre les épaisseurs précitées. La présence de la couche 6 de diffusion est facultative. La taille des pores dans la couche 6 de diffusion peut être adaptée, en modifiant par exemple la concentration en Agarose, afin d'opérer la sélection sur la taille des espèces qui diffusent.

La conception du capteur 2 chimique permet éventuellement d'utiliser les deux faces du substrat, de manière à obtenir deux surfaces 7 libres supérieures permettant de capter les espèces à analyser. La surface de diffusion est alors doublée par rapport au cas où la diffusion est réalisée par une seule surface. La quantité d'espèces capturée par le dispositif 1 est également doublée.

Les avantages du dispositif 1 ainsi formé sont nombreux.

Par exemple, il n'est pas nécessaire de mettre en place une surveillance du dispositif 1 sur une période longue, diminuant les coûts. Au bout de quelques minutes, la quantité d'espèces capturée est suffisante.

De plus, le dispositif 1 se présente sous une forme de tube, aisément manipulable puisqu'il suffit de le plonger dans le milieu à analyser puis de le récupérer à la main, ou fixé sur une corde. Ainsi, l'utilisation du dispositif 1 est tout public, et n'est pas réservée à des opérateurs avertis.

Enfin, la tête 17 chimique, sur laquelle est intégré le capteur 2 chimique, peut être dissociée de la partie électronique du dispositif contenue dans le boîtier 8 de commande. Ainsi, lorsqu'un capteur 2 chimique a été utilisé, il peut être facilement remplacé par un nouveau capteur 2 chimique. Le capteur 2 chimique peut alors être choisi en fonction de l'analyse voulue, des espèces à capturer. Le boîtier 8 de commande étant réutilisable, il est possible d'utiliser le même boîtier 8 de commande pour différentes têtes 17 chimiques, comprenant des capteurs 2 chimiques différents, c'est-à-dire destinés à capturer des espèces chimiques différentes. Par exemple, il a été mentionné une application pour la détection des espèces du mercure. Le dispositif est toutefois adapté pour d'autres applications, par exemple pour détecter d'autres polluants, mais également pour détecter des espèces biologiques. Par exemple, le capteur 2 chimique peut être une puce à ADN.

Grâce aux parties réutilisables et adaptables, les coûts d'utilisation du dispositif 1 sont réduits.

La fabrication de certaines parties telles que le boîtier 8 et les supports 18, 19, 20 en plastique permet également de diminuer les coûts de revient du dispositif 1.

Des variantes du dispositif 1 sont envisageables. En particulier, le dispositif 1 a été précédemment décrit comme comprenant deux parties, le boîtier 8 et le la tête chimique 17, celles-ci étant adaptées pour être connectées l'une à l'autre de manière amovible via l'embout de connexion étanche 10.

Toutefois, dans une variante non représentée, l'embout 8 et la tête chimique 17 sont solidaires l'un de l'autre. En outre, ils ne sont pas adaptés pour être déconnectés l'un de l'autre. Autrement dit, le dispositif 1 se présente sous la forme d'un boîtier unique. Ce boîtier est étanche, à ceci près qu'il présente une zone prévue pour la mise en communication de l'ensemble 12 de capteurs avec l'environnement extérieur.

Dans le cadre de cette variante, le dispositif 1 est dépourvu d'embout de connexion étanche. Les éléments du boîtier 8 et de la tête chimique 17 décrits ci-dessus sont agencés dans une unique enveloppe, à laquelle le capuchon et le bouchon décrits précédemment peuvent être rapportés.

Outre ces éléments, le dispositif 1 comprend un dispositif de communication sans fil (de type « wireless », qui provient de l'anglais et signifie « sans fil ») formant une interface de communication pour les échanges de données entre le dispositif 1 et l'extérieur. Le dispositif de communication est connecté à tout ou partie des éléments du dispositif 1 et autorise ainsi l'émission et la réception de données pour le fonctionnement de ces éléments et la collecte de données.

Dans le cadre de cette variante, l'alimentation contenue dans le dispositif 1, telle que par exemple une batterie, est avantageusement rechargeable en énergie électrique à distance, par exemple par induction.

On remarque que du fait de la configuration du dispositif selon cette variante, l'agencement relatif des éléments qu'il comprend peut être aisément ajusté. Par exemple, dans une réalisation de cette variante, l'alimentation se situe à une extrémité du dispositif, tandis que l'ensemble 12 de capteurs complémentaires et le dispositif de communication se situent à une autre extrémité du dispositif.

Cette variante du dispositif permet de s'affranchir de la présence d'un embout de connexion étanche, qui est un élément onéreux tendant à augmenter substantiellement le coût du dispositif 1.

## Revendications

1. Dispositif (**1**) de captage d'au moins une espèce chimique dans un milieu à analyser comprenant un capteur (**2**) chimique, le capteur (**2**) chimique comprenant :
- un substrat (**4**),
- un support (**5**) fonctionnalisé destiné à capturer et à accumuler l'espèce chimique, et fixé sur une face du substrat (**4**), et
le dispositif comprenant en outre des moyens de diffusion de l'espèce chimique entre le milieu à analyser et
le support (5) fonctionnalisé,
les moyens de diffusion comprenant un système (**3**) générateur de vibrations connecté au capteur (**2**) chimique pour soumettre le capteur (**2**) chimique à des vibrations contrôlées,
le dispositif (**1**) étant **caractérisé en ce que** le système (**3**) générateur de vibrations comprend un micromoteur (**µMOT**) connecté au capteur (**2**) chimique et des moyens de contrôle **(CONT)** du micromoteur **(µMOT**) .

2. Dispositif (**1**) de captage selon la revendication 1, dans lequel les moyens de diffusion comprennent une couche (6) diffusive superposée au support (5) fonctionnalisé.

3. Dispositif (**1**) de captage selon la revendication 2, dans lequel l'épaisseur de la couche (**6**) diffusive est inférieure à 1 micromètre.

4. Dispositif (**1**) de captage selon la revendication 3, dans lequel l'épaisseur de la couche (**6**) diffusive est comprise entre 250 nm et 10 nm.

5. Dispositif **(1)** de captage selon l'une quelconque des revendications 2 à 4, dans lequel la couche **(6)** de diffusion est en gel d'Agarose.

6. Dispositif **(1)** de captage selon l'une quelconque des revendications précédentes, dans lequel l'épaisseur du support (5) fonctionnalisé est inférieure à 1 micromètre.

7. Dispositif **(1)** de captage selon la revendication 6, dans lequel l'épaisseur du support **(5)** fonctionnalisé est inférieure à 1 nm.

8. Dispositif **(1)** de captage selon l'une quelconque des revendications précédentes, dans lequel le substrat **(4)** comprend deux faces opposées sur chacune desquelles est adhérisée un support **(5)** fonctionnalisé.

9. Dispositif de captage selon l'une quelconque des revendications précédentes, dans lequel le système **(3)** générateur de vibrations comprend un capteur **(CAP)** de vibration relié au capteur **(2)** chimique.

10. Dispositif **(1)** de captage selon l'une quelconque des revendications précédentes, dans lequel le micromoteur (**µMOT**) vibre à des fréquences comprises entre 2 et 1000 Hz.

11. Dispositif de captage selon l'une quelconque des revendications précédentes, comprenant d'une part un boîtier **(8)** de commande dans lequel les moyens **(CONT)** de contrôle du micromoteur sont agencés, et d'autre part une tête **(17)** chimique comprenant le capteur **(2)** chimique et le système **(3)** générateur de vibrations, le boîtier **(8)** de commande et la tête **(17)** chimique étant amovibles l'un par rapport à l'autre et comprenant chacun au moins un embout **(10, 21)** de connexion pour les assembler l'un avec l'autre.

12. Dispositif de captage selon l'une quelconque des revendications 1 à 10, le dispositif de captage se présentant sous la forme d'un boîtier unique.

13. Dispositif (**1**) de captage selon l'une quelconque des revendications 1 à 12, dans lequel le capteur (2) est fabriqué par un procédé comprenant :
- le décapage d'au moins une face d'une plaque formant le substrat **(4).**
- l' adhérisation du support **(5)** fonctionnalisé sur la face décapée du substrat **(4),** le support **(5)** fonctionnalisé ayant une épaisseur inférieure à 1 micromètre ;
- le dépôt par spin-coating, ou dip coating d'une couche **(6)** diffusive sur le support **(5)** fonctionnalisé, la couche (6) diffusive ayant une épaisseur inférieure à 1 micromètre.

14. Procédé de détermination de la concentration d'au moins une espèce chimique dans un milieu à analyser au moyen d'un dispositif (1) de captage selon l'une quelconque des revendications précédentes, comprenant :
- mettre le capteur (2) chimique en contact avec le milieu à analyser ;
contrôler le micromoteur (µMOT), en utilisant les moyens de contrôle (CONT), pour soumettre le capteur (2) chimique à des vibrations de fréquences contrôlées par le système (3) générateur de vibrations ;
- capter l'espèce chimique à analyser par le support (5) fonctionnalisé par diffusion entre le milieu à analyser et le support (5) fonctionnalisé ;
- au bout d'une durée déterminée, récupération du capteur (2) chimique hors du milieu à analyser ;
- extraction de l'espèce chimique piégée dans le capteur (2) chimique ;
- en utilisant le principe de diffusion et la durée déterminée, détermination de la concentration de l'espèce chimique à analyser.

15. Procédé selon la revendication 14, dans lequel la fréquence des vibrations est comprise entre 2 et 1000 Hz.

16. Procédé selon la revendication 15, dans lequel la fréquence des vibrations est comprise entre 10 et 600 Hz.

17. Procédé selon l'une des revendications 14 à 16, dans lequel au moins une espèce chimique est le méthylmercure.

## Patentansprüche

1. Vorrichtung **(1)** zum Erfassen wenigstens einer chemischen Spezies in einem zu analysierenden Medium mit einem chemischen Sensor **(2),** wobei der chemische Sensor **(2)** umfasst:
- ein Substrat **(4),**
- einen funktionalisierten Träger **(5),** der dazu bestimmt ist, die chemische Spezies zu erfassen und zu akkumulieren, und der an einer Seite des Substrats **(4)** befestigt ist, und
wobei die Vorrichtung ferner Mittel zum Diffundieren der chemischen Spezies zwischen dem zu analysierenden Medium und dem funktionalisierten Träger **(5)** umfasst,
wobei die Diffusionsmittel ein Vibrationserzeugungssystem **(3)** umfassen, das mit dem chemischen Sensor **(2)** verbunden ist, um den chemischen Sensor **(2)** kontrollierten Vibrationen auszusetzen,
wobei die Vorrichtung **(1) dadurch gekennzeichnet ist, dass** das Vibrationserzeugungssystem **(3)** einen Mikromotor **(µMOT)** umfasst, der mit dem chemischen Sensor **(2)** verbunden ist, und Mittel **(CONT)** zum Steuern des Mikromotors (**µMOT**)

2. Erfassungsvorrichtung **(1)** nach Anspruch 1, wobei die Diffusionsmittel eine Diffusionsschicht **(6)** umfassen, die auf dem funktionalisierten Träger **(5)** aufgebracht ist.

3. Erfassungsvorrichtung **(1)** nach Anspruch 2, wobei die Dicke der Diffusionsschicht **(6)** weniger als 1 Mikrometer beträgt.

4. Erfassungsvorrichtung **(1)** nach Anspruch 3, wobei die Dicke der Diffusionsschicht **(6)** zwischen 250 nm und 10 nm beträgt.

5. Erfassungsvorrichtung **(1)** nach einem der Ansprüche 2 bis 4, wobei die Diffusionsschicht **(6)** aus Agarose-Gel besteht.

6. Erfassungsvorrichtung **(1)** nach einem der vorhergehenden Ansprüche, wobei die Dicke des funktionalisierten Trägers **(5)** weniger als 1 Mikrometer beträgt.

7. Erfassungsvorrichtung **(1)** nach Anspruch 6, wobei die Dicke des funktionalisierten Trägers **(5)** weniger als 1 nm beträgt.

8. Erfassungsvorrichtung **(1)** nach einem der vorhergehenden Ansprüche, wobei das Substrat **(4)** zwei gegenüberliegende Seiten aufweist, an denen jeweils ein funktionalisierter Träger **(5)** angehaftet ist.

9. Erfassungsvorrichtung nach einem der vorhergehenden Ansprüche, wobei das Vibrationserzeugungssystem **(3)** einen mit dem chemischen Sensor **(2)** verbundenen Vibrationssensor **(CAP)** umfasst.

10. Erfassungsvorrichtung **(1)** nach einem der vorhergehenden Ansprüche, wobei der Mikromotor **(µMOT)** mit Frequenzen zwischen 2 und 1000 Hz vibriert.

11. Erfassungsvorrichtung nach einem der vorhergehenden Ansprüche, umfassend einerseits ein Steuergehäuse **(8),** in dem die Steuermittel **(CONT)** des Mikromotors angeordnet sind, und andererseits ein chemisches Kopfstück **(17),** das den chemischen Sensor **(2)** und das Vibrationserzeugungssystem **(3)** umfasst, wobei das Steuergehäuse **(8)** und das chemische Kopfstück **(17)** in Bezug zueinander abnehmbar sind und jeweils wenigstens ein Verbindungsendstück **(10, 21)** zu ihrem Zusammenbau umfassen.

12. Erfassungsvorrichtung nach einem der Ansprüche 1 bis 10, wobei die Sensoreinrichtung die Form eines einzelnen Gehäuses aufweist.

13. Erfassungsvorrichtung **(1)** nach einem der Ansprüche 1 bis 12, wobei der Sensor **(2)** durch ein Verfahren hergestellt wird, umfassend:
- Ätzen wenigstens einer Seite einer Platte, die das Substrat **(4)** bildet,
- Anhaften des funktionalisierten Trägers **(5)** an der geätzten Fläche des Substrats **(4),** wobei der funktionalisierte Träger **(5)** eine Dicke von weniger als 1 Mikrometer aufweist,
- Ablagerung durch Schleuderbeschichtung (spin-coating) oder Tauchbeschichtung (dip coating) einer Diffusionsschicht **(6)** auf dem funktionalisierten Träger **(5),** wobei die Diffusionsschicht **(6)** eine Dicke von weniger als 1 Mikrometer aufweist.

14. Verfahren zur Bestimmung der Konzentration wenigstens einer chemischen Spezies in einem zu analysierenden Medium mittels einer Erfassungsvorrichtung **(1)** nach einem der vorhergehenden Ansprüche, umfassend:
- In-Kontakt-bringen des chemischen Sensors **(2)** mit dem zu analysierenden Medium,
- Steuerung des Mikromotors **(µMOT)** unter Verwendung der Steuermittel **(CONT),** um den chemischen Sensor **(2)** Vibrationen zu unterziehen, deren Frequenz durch das Vibrationserzeugungssystem **(3)** gesteuert wird,
- Erfassen der zu analysierenden chemischen Spezies durch den funktionalisierten Träger **(5)** durch Diffusion zwischen dem zu analysierenden Medium und dem funktionalisierten Träger **(5),**
- nach einer bestimmten Zeitdauer Wiedergewinnung des chemischen Sensors **(2)** aus dem zu analysierenden Medium,
- Extraktion der im chemischen Sensor **(2)** eingeschlossenen chemischen Spezies,
- unter Verwendung des Diffusionsprinzips und der bestimmten Zeitdauer, Bestimmung der Konzentration der zu analysierenden chemischen Spezies.

15. Verfahren nach Anspruch 14, wobei die Frequenz der Vibrationen zwischen 2 und 1000 Hz liegt.

16. Verfahren nach Anspruch 15, wobei die Frequenz der Vibrationen zwischen 10 und 600 Hz liegt.

17. Verfahren nach einem der Ansprüche 14 bis 16, wobei wenigstens eine chemische Spezies Methylquecksilber ist.

## Claims

1. Device (1) for detecting at least one chemical species in a medium to be analysed comprising a chemical sensor (2), the chemical sensor (2) comprising:
- a substrate (4),
- a functionalised carrier (5) intended for capturing and accumulating the chemical species and attached to a side of the substrate (4), and
the device also comprising means for diffusion of the chemical species between the medium to be analysed and the functionalised carrier (5),
the diffusion means comprising a vibration generating system (3) connected to the chemical sensor (2) in order to subject the chemical sensor (2) to controlled vibrations, the device (1) being **characterised in that** the vibration generating system (3) comprises a micro-motor (µMOT) connected to the chemical sensor (2) and to control means (CONT) of the micro-motor (µMOT).

2. Detection device (1) according to claim 1, wherein the diffusion means comprise a diffusive layer (6) superimposed on the functionalised carrier (5).

3. Detection device (1) according to claim 2, wherein the thickness of the diffusive layer (6) is preferably less than 1 micrometre.

4. Detection device (1) according to claim 3, wherein the thickness of the diffusive layer (6) lies in the range 250 nm to 10 nm.

5. Detection device (1) according to any of claims 2 to 4, wherein the diffusion layer (6) is made from Agarose gel.

6. Detection device (1) according to any of the previous claims, wherein the thickness of the functionalised carrier (5) is less than 1 micrometer.

7. Detection device (1) according to claim 6, wherein the thickness of the functionalised carrier (5) is less than 1 nm.

8. Detection device (1) according to any of the previous claims, wherein the substrate (4) comprises two opposite sides, on each of which is adhered a functionalised carrier (5).

9. Detection device according to any of the previous claims, wherein the vibration generating system (3) comprises a vibration sensor (CAP) connected to the chemical sensor (2).

10. Detection device (1) according to any of the previous claims, wherein the micro-motor (µMOT) vibrates at frequencies between 2 and 1000 Hz.

11. Detection device according to any of the previous claims, comprising on the one hand a control unit (8) in which the control means (CONT) of the micro-motor are arranged, and on the other hand comprising a chemical head (17) comprising the chemical sensor (2) and the vibration generating system (3), the control unit (8) and the chemical head (17) being capable of being removed from each other and each comprising at least one connection tip (10, 21) for the assembly thereof with each other.

12. Detection device according to any of claims 1 to 10, the detection device being present in the form of a single unit.

13. Detection device according to any of claims 1 to 12, wherein the sensor (2) is manufactured by a method comprising:
- etching at least one side of a plate forming the substrate (4),
- adhering the functionalised carrier (5) to the etched side of the substrate (4), the functionalised carrier (5) having a thickness of less than 1 micrometre;
- depositing, by spin-coating or dip-coating, a diffusive layer (6) onto the functionalised carrier (5), the diffusive layer (6) having a thickness of less than 1 micrometre.

14. Method for determining the concentration of at least one chemical species in a medium to be analysed by means of a detection device (1) according to any of the previous claims, comprising:
- placing the chemical sensor (2) in contact with the medium to be analysed;
- controlling the micro-motor (µMOT), by using the control means (CONT) in order to subject the chemical sensor (2) to vibrations at frequencies controlled by the vibration generating system (3);
- detecting the chemical species to be analysed by the functionalised carrier (5) by diffusion between the medium to be analysed and the functionalised carrier (5)
- after a determined duration, retrieving the chemical sensor (2) out of the medium to be analysed;
- extracting the chemical species trapped in the chemical sensor (2);
- by using the diffusion principle and the determined duration, determining the concentration of the chemical species to be analysed.

15. Method according to claim 14, wherein the frequency of the vibrations lies in the range 2 to 1000 Hz.

16. Method according to claim 15, wherein the frequency of the vibrations lies in the range 10 to 600 Hz.

17. Method according to one of claims 14 to 16, wherein at least one chemical species is methylmercury.
